# EUROPÄISCHE PATENTSCHRIFT

(11) **EP 3 257 474 B1**
(45) Veröffentlichungstag und Bekanntmachung des Hinweises auf die Patenterteilung: **18.09.2019**
(21) Anmeldenummer: 16174177.2
(22) Anmeldetag: 13.06.2016
(51) Int. Cl.: A61F 2/28, A61F 2/30

(54) **ENDOPROTHESE FÜR EINEN RÖHRENKNOCHEN**
ENDOPROSTHESIS FOR A TUBULAR BONE
ENDOPROTHESE POUR UN OS LONG

(43) Veröffentlichungstag der Anmeldung: 20.12.2017
(73) Patentinhaber: Boesch, René Daniel, 6965 Cadro (CH)
(72) Erfinder: Boesch, René Daniel, 6965 Cadro (CH)
(74) Vertreter: Schaad, Balass, Menzl & Partner AG

(56) Entgegenhaltungen:
- EP-A1- 1 407 727
- EP-A1- 2 759 281
- WO-A2-2010/082183
- US-A- 5 489 311
- US-B1- 6 709 466

## Beschreibung

Die Erfindung betrifft eine Endoprothese für einen Röhrenknochen mit einem Schaft und einem damit verbindbaren Kopfteil sowie ein Endoprothese-Set enthaltend eine Anzahl von unterschiedlichen Schäften und Kopfteilen. Die erfindungsgemässe Endoprothese eignet sich insbesondere als Implantat für den Femurstumpf bei Personen, die eine Beinprothese tragen.

Üblicherweise wird in solchen Fällen ein Trichter bzw. Schaft über den Oberschenkelstumpf des zu versorgenden Patienten gezogen, an dem die weiteren Teile der Prothese wie Kniegelenk-, Unterschenkel- und Fussteil angebracht sind.

Bei herkömmlichen Prothesen stützt sich bei einer Belastung der sogenannte Tuber auf den oberen Rand des Prothesenschaftes ab, während der Beinstumpf im Prothesenschaft belastungsfrei bleibt. Der Tuber ist aber an sich kein Stützknochen und zur Aufnahme von Belastungen nicht geeignet. Wegen dieser Abstützung des Tubers auf dem Prothesenschaft treten daher bei Prothesenträgern häufig Schmerzen auf. Als Folge davon scheuen sich Personen mit einer Prothese oft, die Prothese zu belasten, und verlagern stattdessen das Körpergewicht möglichst auf das andere, gesunde Bein. Ausserdem ist die Abstützung des Tubers auf dem Prothesenschaft auch in Bezug auf die Stabilität nicht optimal.

Ein grosses Problem bei solchen herkömmlichen Prothesen ist, wie die Belastungskräfte über das Gewebe in den Femurstumpf eingeleitet werden können. Zwischen dem Femurstumpf und dem Äusseren des Oberschenkelstumpfes befindet sich nämlich Muskelgewebe, welches bei einer Bewegung ständig hohen Belastungen ausgesetzt ist, ohne dass das Wahrnehmungsgefühl hierdurch gefördert wird in Richtung auf den natürlichen Gehkontakt.

Um die Kräfteübertragung vom Tuber auf den Femurknochen zu verschieben, wurden bereits verschiedene Implantate für den Femurstumpf entwickelt:
Aus DE 43 38 746 und DE 31 25 268 sowie EP 2 759 281 sind Femurstumpfimplantate bekannt, welche das angesprochene Problem dadurch versuchen zu lösen, dass in den Femurstumpf ein Stielteil gesetzt wird, das auf Dauer im Knochenkanal fixiert wird. Zum anderen Ende hin weisen die Implantate pilzkopfartig vergrösserte Kopfenden auf, welche die Belastungskräfte auf grössere Flächen verteilen sollen. Diese Art der Versorgung verspricht einen besseren Tragekomfort gegenüber den bislang üblichen Versorgungsmöglichkeiten. Wenn auch das Wahrnehmungsgefühl beim Patienten mit dem natürlichen Wahrnehmungsgefühl vor der Amputation praktisch nichts gemein hat.

Eine erste Weiterentwicklung davon ist in DE 198 57 907 offenbart, wo eine Oberschenkelstumpf-Endoprothese mit einem proximalen Stielteil zum Einsetzen in den Femurstumpf verwendet wird, der mit einer offenmaschigen, dreidimensionalen Raumnetzstruktur bedeckt ist. An seinem distalen Ende weist der Stielteil einen konischen Adapter auf, mittels dessen ein Kondylenersatz, der der Form natürlicher Kondylen eines Kniegelenks nachgebildet ist, an den Stielteil gekoppelt ist. Durch diese Anordnung soll das Wahrnehmungsgefühl des Patienten gesteigert und das Gehverhalten so als natürlicher empfunden werden.

Darauf aufbauend beschreibt WO 01/05335 eine Oberschenkelstumpf-Endoprothese, bei der mit dem Kondylenersatz eine keilförmige Unterlage verbunden ist, welche die natürliche Valgität, d.h. also die Winkelstellung des Femurstumpfes in Richtung medial, wiederherstellt. Hierdurch soll die Kräfteverteilung denjenigen in einem gesunden Bein nachempfunden werden.

Eine weitere Endoprothese für einen Femurstumpf ist aus US 2007/0150070 bekannt. Dabei wird ein Implantat in den Knochenstumpf eingeführt und mit einem belastungsverteilenden, deckelförmigen Adapter verbunden, der sowohl das Implantat als auch das untere Ende des Knochenstumpfes umgreift. Am Adapter ist mithilfe einer Schraube eine Lastträgerplatte befestigt.

Ausgehend von diesen bekannten Endoprothesen hat sich die Aufgabe gestellt, die Kräfteverteilung und Stabilität - und damit auch das Wahrnehmungsgefühl des Patientenweiter zu verbessern. Gleichzeitig soll eine Endoprothese zu Verfügung gestellt werden, die sich zuverlässig und kostengünstig herstellen und auf einfache Weise an den jeweiligen Patienten anpassen lässt.

Diese Aufgaben werden gelöst durch die Endoprothese gemäss Anspruch 1 sowie das Endoprothese-Set gemäss Anspruch 14. Bevorzugte Ausführungsformen sind Gegenstand der abhängigen Ansprüche.

Die vorliegende Erfindung betrifft einen Endoprothese für einen Röhrenknochen mit einer Knochenwand und einer Markhöhle, insbesondere für einen Femurstumpf. Die Endoprothese weist einen wenigstens annähernd geraden Schaft mit einem proximalen Implantatabschnitt und einem distalen Kopfabschnitt auf, der eine Längsrichtung L definiert und dazu bestimmt ist, in die Markhöhle des Röhrenknochens eingesetzt zu werden. Der proximale Implantatabschnitt weist ein proximales Ende auf und der distale Kopfabschnitt ein distales Ende. Die Endoprothese weist weiter einen am distalen Kopfabschnitt des Schafts befestigbaren Kopfteil mit einer am Kopfteil angeformten Stützfläche auf, die dazu bestimmt ist, im implantierten Zustand der Endoprothese eine distale Endfläche des Röhrenknochens zu stützen.

Die erfindungsgemässe Endoprothese ist dadurch gekennzeichnet, dass der distale Kopfabschnitt des Schafts eine zum distalen Ende hin offene, distale Ausnehmung mit einem Boden aufweist und der Kopfteil einen gegenüber der Stützfläche vorstehenden, integralen Verbindungszapfen mit einer Basis und einem freien Zapfenende aufweist. Der Verbindungszapfen ist dazu bestimmt, zur Verbindung von Kopfteil und Schaft mit dem freien Ende voran in die distale Ausnehmung des Schafts eingesteckt zu werden.

Die erfindungsgemässe Endoprothese ist besonders gut dafür geeignet, in einen Femurstumpf eingesetzt zu werden. Dazu wird der Schaft mit dem proximalen Implantatabschnitt voran in die Markhöhle des Knochens eingeführt. Im implantierten Zustand liegt die Stützfläche des Kopfteils an der distalen Endfläche bzw. am distalen Ende des Knochens an und erlaubt so, Belastungen von einer äusseren Beinprothese über den Kopfteil auf den Femurknochen zu übertragen. Dadurch wird eine Entlastung des Tubers erreicht und so Schmerzen verhindert. Gleichzeitig wird auch das Wahrnehmungsgefühl beim Gehen verbessert.

Durch den Kopfteil, der vorzugsweise grossflächig ist, werden die Belastungskräfte optimal vom Röhrenknochen auf die Endoprothese verteilt und der Druck auf die äussere Beinprothese verkleinert. Ausserdem vereinfacht der Kopfteil auch die Befestigung der äusseren Beinprothese am Oberschenkelstumpf.

Durch die Verwendung von separatem Schaft und Kopfteil wird eine grosse Flexibilität bei der präoperativen Planung erreicht: Die Endoprothese kann durch Ausweil eines geeigneten Schafts und eines geeigneten Kopfteils optimal an die Anatomie eines Patienten angepasst werden. Dies betrifft insbesondere die Länge und den Durchmesser des Schafts, die Neigung des Kopfteils gegenüber der Längsachse des Schafts, sowie die Grösse und Form des Kopfteils.

Zur Verbindung von Schaft und Kopfteil wird der Verbindungszapfen des Kopfteils mit dem freien Zapfenende voran in die distale Ausnehmung des Schafts eingesteckt. Vorzugsweise wird der Kopfteil zusätzlich mithilfe einer Schraube am Schaft befestigt. Im implantierten Zustand ist die distale Ausnehmung also zum Kopfteil hin und somit bei einem stehenden Patienten nach unten offen. Dies hat den Vorteil, dass allfälliges Wundwasser nicht in die Ausnehmung hineinfliessen und sich dort ansammeln kann.

Die erfindungsgemässe Endoprothese besteht vorzugsweise zumindest hauptsächlich aus Titan oder einer Titanlegierung, insbesondere aus TiAl6V4 oder TiAl6Nb7. Alternativ kann auch eine Kobalt-Chrom-Molybdän-Legierung (CoCrMo) verwendet werden. In besonderen Fällen, beispielsweise bei einer Allergie, können aber auch andere aus dem Stand der Technik bekannte Materialien verwendet werden. Besonders bevorzugt bestehen Schaft und Kopfteil aus dem selben Material.

Gemäss einer bevorzugten Ausführungsform weist die Ausnehmung des Schafts einen Passsitzabschnitt mit einer innen liegenden Mantelfläche auf und der Verbindungszapfen des Kopfteils einen korrespondierenden Passsitzabschnitt mit einer aussen liegenden Mantelfläche. Dabei sind die beiden Mantelflächen dazu bestimmt, im montierten Zustand aneinander anzuliegen, um einen Passsitz auszubilden. Durch Vorsehen zweier korrespondierender Mantelflächen wird ein Spalt zwischen Kopfteil und Schaft verhindert.

Gemäss einer besonders bevorzugten Ausführungsform ist die innen liegende Mantelfläche der Ausnehmung des Schaftes in Richtung zum Boden der Ausnehmung konisch verjüngt ausgebildet und die aussen liegende Mantelfläche des Verbindungszapfens in Richtung zum freien Ende des Verbindungszapfens entsprechend konisch verjüngt ausgebildet. In diesem Fall wird der Passsitz als Kegelsitz ausgebildet. Weiter ist es bevorzugt, dass das freie Zapfenende des Kopfteils im verbundenen Zustand den Boden der Ausnehmung des Schaftes nicht berührt.

Gemäss einer besonders bevorzugten Ausführungsform sind die innen liegende Mantelfläche und die aussen liegende Mantelfläche gegenüber der Längsrichtung L um einen Winkel von 1° bis 6°, vorzugsweise 3°, geneigt, was einem Konuswinkel von 2° bis 12°, vorzugsweise von 6°, entspricht. Dies ergibt einen optimalen Kegelsitz zwischen den beiden Mantelflächen.

Gemäss einer bevorzugten Ausführungsform weist die erfindungsgemässe Endoprothese zwischen dem Passsitzabschnitt des Kopfteils und der Basis des Verbindungszapfens einen konusförmigen Abschnitt auf. Dieser konusförmige Abschnitt ist im implantierten Zustand üblicherweise ausserhalb der distalen Ausnehmung des Schafts aber innerhalb der Markhöhle des Röhrenknochens angeordnet. Vorzugsweise weist er einen grösseren Neigungswinkel gegenüber der Längsrichtung L auf als der Passsitzabschnitt des Kopfteils. Er dient dazu, die Kräfteübertragung weiter zu verbessern.

Gemäss einer bevorzugten Ausführungsform weist die erfindungsgemässe Endoprothese ein Befestigungselement auf zur Befestigung des Kopfteils am Schaft. Dabei handelt es sich vorzugsweise um eine Schraube. Dies erlaubt eine einfache und sichere Befestigung des Kopfteils am Schaft, die im Bedarfsfall auch auf einfache Weise wieder lösbar ist.

Besonders bevorzugt weist der Kopfteil eine in Längsrichtung L verlaufende, durchgehende Öffnung mit einer im Bereich des freien Zapfenendes angeordneten Schulter auf und der Schaft eine vom Boden der distalen Ausnehmung ausgehende, in Längsrichtung L verlaufende, sacklochförmige Vertiefung. Öffnung und sacklochförmige Vertiefung sollten dabei so ausgestaltet sein, dass das Befestigungselement in die Öffnung des Kopfteils einsetzbar ist und im montierten Zustand mit der sacklochförmigen Vertiefung zusammenwirkt. Vorzugsweise wird als Befestigungselement eine Schraube verwendet, die in ein Innengewinde der sacklochförmigen Vertiefung eingreift.

Bei der obigen bevorzugten Ausführungsform umfasst die erfindungsgemässe Endoprothese zusätzlich vorzugsweise eine Abdeckhülse, welche dazu bestimmt ist, im montierten Zustand in die Öffnung des Kopfteils eingesetzt zu sein und das Befestigungselement vom umliegenden Gewebe abzutrennen.

Üblicherweise werden zunächst Schaft und Kopfteil miteinander zur erfindungsgemässen Endoprothese verbunden und anschliessend in den Röhrenknochen eingesetzt.

Die Verankerung des Schafts der erfindungsgemässen Endoprothese im Knochen wird vorzugsweise durch Einzementieren bewerkstelligt. Wenn Grösse und Form des Schafts optimal an die Markhöhle angepasst sind, so kann der Schaft auch ohne Zement durch reinen Kraftschluss, also beispielsweise durch Presssitz, befestigt werden.

Gemäss einer bevorzugten Ausführungsform ist der proximale Implantatabschnitt in Richtung zum proximalen Ende sich verjüngend ausgebildet. Dies erlaubt ein einfaches Einführen des Schafts in die Markhöhle des Röhrenknochens und eine sichere Verankerung darin. Insbesondere wird durch einen gegenüber dem proximalen Ende erweiterten Abschnitt im Bereich des distalen Endes ein möglichst lückenloses Anliegen des Schafts an der Innenoberfläche der Markhöhle im Endbereich des Knochens gewährleistet.

Besonders bevorzugt ist der distale Kopfabschnitt des Schafts kreiszylinderförmig, so dass die auf die Knochenwand wirkende Spannung in radialer Richtung minimiert werden können. Dabei kann die Markhöhle im Bereich des distalen Kopfabschnitts leicht aufgeweitet sein, beispielsweise durch Bohren.

Besonders bevorzugt ist der proximale Implantatabschnitt mit mindestens einem Verdrehsicherungselement versehen, insbesondere mit einer oder mehreren in Längsrichtung verlaufenden Vertiefungen, Nuten, Anfasungen oder seitlich abstehenden Rippen. Dies verhindert, dass sich der Schaft im implantierten Zustand, beispielsweise unter Belastung, relativ zum Knochen dreht, was auch ein Drehen des Kopfteils bewirken würde. Dies ist insbesondere dann wichtig, wenn der Kopfteil nicht rotationssymmetrisch ausgestaltet ist. Zudem ist ein Verdrehsicherungselement insbesondere dann vorteilhaft, wenn der Schaft im Knochen nicht mittels Zement befestigt wird.

Gemäss einer bevorzugten Ausführungsform erstreckt sich die Stützfläche des Kopfteils wenigstens annährend ringförmig von der Basis des Verbindungszapfens radial auswärts. Besonders bevorzugt ist sie zudem mit einer gerippten, vorzugsweise metallischen, Oberflächenstruktur versehen. Dies gewährleistet eine sichere und stabile Abstützung des Knochens durch die Stützfläche.

Die Ausrichtung der Stützfläche sollte möglichst genau an den Knochenstumpf angepasst sein, so dass die distale Endfläche des Knochenstumpfs im implantierten Zustand möglichst vollständig an der Stützfläche anliegt. Vorzugsweise verläuft die Stützfläche wenigstens annähernd rechtwinklig zur Längsachse des Knochenstumpfes und damit auch wenigstens annähernd rechtwinklig zur Längsrichtung L des Schafts.

Auch die Form, Grösse und Ausrichtung des Kopfteils sollte möglichst genau auf die anatomischen Gegebenheiten des jeweiligen Patienten abgestimmt werden.

Gemäss einer bevorzugten Ausführungsform weist der Kopfteil einen im Wesentlichen quaderförmigen Körper mit abgerundeten Ecken und Kanten mit möglichst grossen Radien auf, der gegenüber der Längsrichtung L geneigt ist. Dieser Körper ist vorzugsweise über einen sich verjüngenden Übergangsabschnitt integral mit der Stützfläche verbunden.

Gemäss einer bevorzugten Ausführungsform weist der Kopfteil auf der dem Verbindungszapfen abgewandten Seite eine in radialer Richtung über die Stützfläche hinausragende Tragfläche auf. Dadurch ergibt sich für die äussere Beinprothese eine grössere Auflagefläche, über welche die auf die Beinprothese wirkenden Kräfte auf den Femurstumpf übertragen werden können. Die Tragfläche ist vorzugsweise wenigstens annähernd rechteckig mit gerundeten Ecken und/oder vorzugsweise wenigstens annähernd eben.

Besonders bevorzugt ist diese Tragfläche gegenüber der Stützfläche um einen Winkel von 4 bis 12°, besonders bevorzugt um einen Winkel von 6 bis 9°, geneigt. Auch durch die Neigung der Tragfläche gegenüber der Stützfläche wird eine natürlichere Wahrnehmung für den Patienten bewirkt. Insbesondere wird dadurch eine ähnliche Kräfteübertragung wie beim natürlichen Femurknochen erreicht.

Je nach Anatomie des Patienten kann ein grösserer oder kleinerer Neigungswinkel bevorzugt sein. Dies ist bei der präoperativen Planung und insbesondere bei der Wahl des Kopfteils unbedingt zu beachten. Typischerweise sollte der Winkel zwischen der Tragfläche und der Stützfläche bei einem gross gewachsenen Patienten ca. 6° betragen, während für einen kleineren Patienten ein Winkel von ca. 9° ideal ist.

Die vorliegende Erfindung betrifft weiter ein Endoprothese-Set enthaltend eine Anzahl von unterschiedlichen Schäften und Kopfteilen mit den oben beschriebenen Merkmalen. Innerhalb des erfindungsgemässen Endoprothese-Sets sind die Ausnehmungen und Verbindungszapfen derart geformt, dass jeder Schaft mit jedem Kopfteil verbunden werden kann.

Dies erlaubt es dem behandelnden Arzt, aus einer Anzahl von Schäften und Kopfteilen jeweils denjenigen Schaft und denjenigen Kopfteil auszuwählen, welche optimal an die anatomischen Verhältnisse des jeweiligen Patienten angepasst sind. Dadurch wird die präoperative Planung signifikant vereinfacht.

Innerhalb des Sets können insbesondere Schäfte vorhanden sein, die eine unterschiedliche Länge und/oder einen unterschiedlichen Durchmesser aufweisen. So kann der Schaft optimal an die Grösse und Form der Markhöhle angepasst werden.

Innerhalb des Sets können insbesondere Kopfteile vorhanden sein, die eine unterschiedliche Dicke und/oder eine unterschiedliche Grösse und/oder eine unterschiedliche Form und/oder eine unterschiedliche Neigung zwischen Stützfläche und Tragfläche aufweisen. So kann durch die Wahl des richtigen Kopfteils eine optimale Anpassung an die Anatomie des Patienten vorgenommen werden.

Daneben können innerhalb des Sets beispielweise auch Schäfte und/oder Kopfteile mit unterschiedlichen Oberflächen vorhanden sein.

Bevorzugte Ausführungsformen der erfindungsgemässen Endoprothese sind auch bevorzugte Ausführungsformen des erfindungsgemässen Endoprothese-Sets.

Die vorliegende Erfindung soll anhand einer in der Zeichnung gezeigten, bevorzugten Ausführungsform weiter veranschaulicht werden.

Es zeigt, rein schematisch:
- Fig. 1: in einer Explosionsdarstellung eine bevorzugte Ausführungsform einer erfindungsgemässen Endoprothese mit einem Schaft, einem Kopfteil und einer Verbindungsschraube;
- Fig. 2a: eine Seitenansicht des Schafts aus Fig. 1;
- Fig. 2b: einen Längsschnitt durch den Schaft aus Fig. 2a;
- Fig. 3a: eine perspektivische Draufsicht auf den Kopfteil aus Fig. 1;
- Fig. 3b: eine Längsseitenansicht des Kopfteils aus Fig. 3a;
- Fig. 3c: eine Untersicht des Kopfteils aus Fig. 3a;
- Fig. 3d: eine Querseitenansicht des Kopfteils aus Fig. 3a; und
- Fig. 4: eine Seitenansicht der Endoprothese aus Fig. 1 im implantierten Zustand in einem teiltransparent dargestellten Femurstumpf.

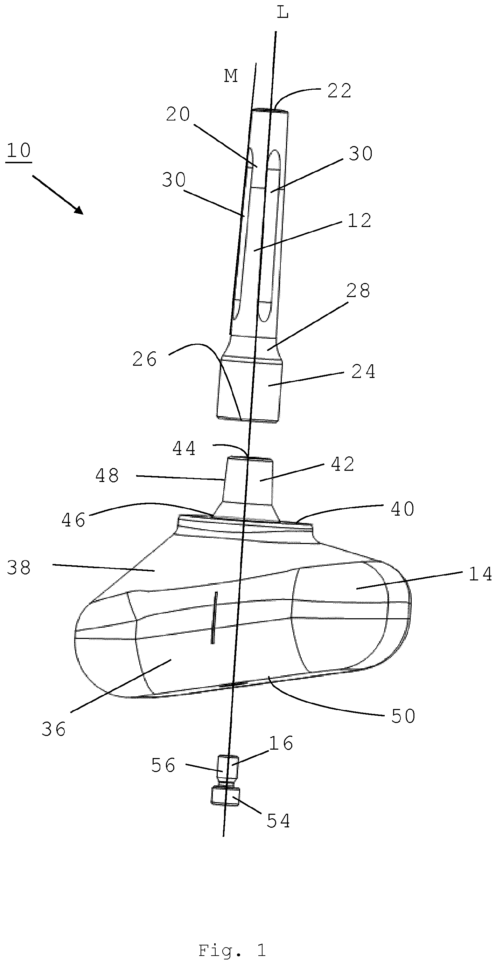

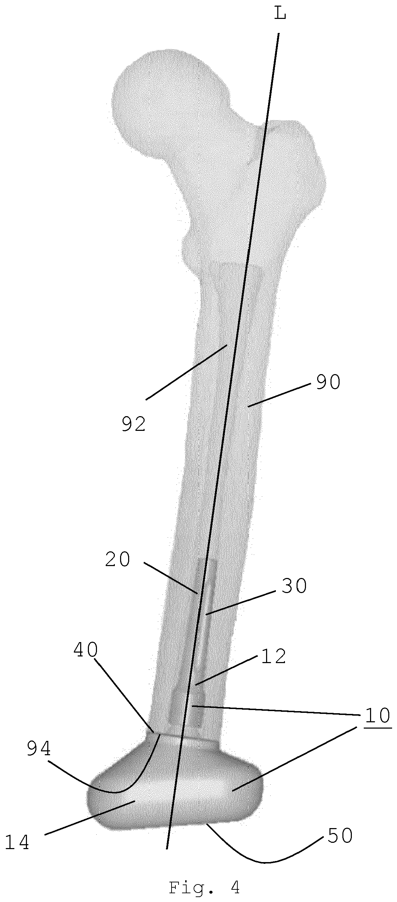

Figur 1 zeigt in Form einer Explosionsdarstellung eine schematische Seitenansicht einer Endoprothese 10, welche dazu bestimmt ist, in die Markhöhle eines Femurstumpfes (vgl. Fig. 4) eingeführt zu werden. Die Endoprothese 10 umfasst einen länglichen Schaft 12, einen Kopfteil 14 und eine Schraube 16.

Der Schaft 12 besteht vorzugsweise aus Titan oder einer Titanlegierung. Er weist einen kreisförmigen Querschnitt auf und definiert eine Längsrichtung L. Er weist einen konischen proximalen Implantatabschnitt 20 mit einem proximalen Ende 22 und einen wenigstens annähernd kreiszylinderförmigen distalen Kopfabschnitt 24 mit einem distalen Ende 26 auf. Zwischen dem proximalen Implantatabschnitt 20 und dem distalen Kopfabschnitt 24 ist ein trompetenartig verjüngter Übergangsabschnitt 28 angeordnet.

Der Schaft 12 ist dazu bestimmt, in der Längsrichtung L mit dem proximalen Ende 22 voran in die Markhöhle eines Femurstumpfes (vgl. Fig. 4) eingeführt zu werden. Der proximale Implantatabschnitt 20 weist einen geringeren Durchmesser auf als der distale Kopfabschnitt 24, während der Übergangsabschnitt 28 vom distalen Kopfabschnitt 24 zum proximalen Implantatabschnitt 20 trompetenartig verjüngt ist.

Der proximale Implantatabschnitt 20 ist zum proximalen Ende 22 hin leicht konisch verjüngt. Zwischen dem proximalen Ende 22 und dem Übergangsabschnitt 28 weist der proximale Implantatabschnitt 20 mehrere längliche Nuten 30 auf, die als Verdrehsicherungselemente dienen. Bei der gezeigten ausführungsform sind vier längliche in Nuten 30 vorhanden, die in Längsrichtung L verlaufen und in Umfangsrichtung regelmässig verteilt sind. Die Nuten 30 erstrecken sich über einen Grossteil der Länge des proximalen Implantatabschnitts 20.

Der Übergangsabschnitt 28 ist im Wesentlichen trompetenförmig ausgebildet. Er dient dazu, die Kräfteübertragung vom Schaft über den Verbindungszapfen auf den Kopfteil zu verbessern.

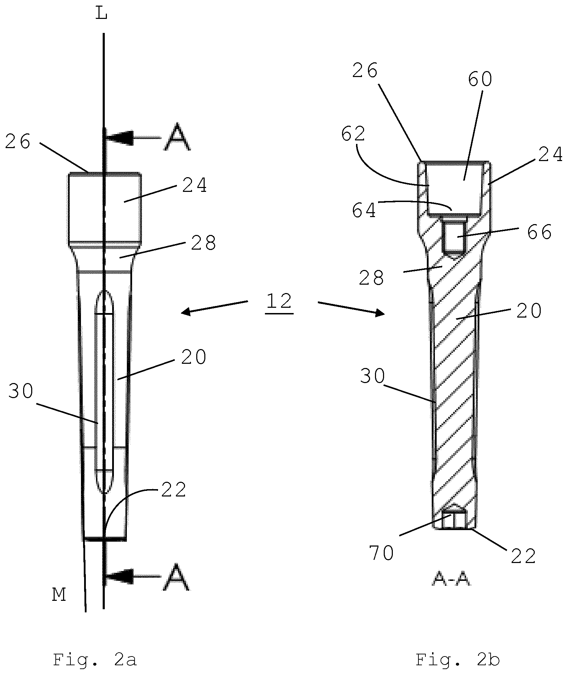

Der distale Kopfabschnitt 24 ist im Wesentlichen kreiszylinderförmig, wodurch die radiale Spannung auf die umgebende Knochenwand minimiert werden. Ausserdem kann die Markhöhle im Bereich des distale Kopfabschnitt 24 aufgeweitet sein (vgl. Fig. 4). Der distale Kopfabschnitt 24 weist eine zum distalen Ende 26 hin offene, distale Ausnehmung auf (vgl. Fig. 2b).

Der Durchmesser des distalen Kopfabschnitts 24 ist vorliegend fast doppelt so gross wie der Durchmesser des proximalen Implantatabschnitts 20 am proximalen Ende 22. Je nach Anatomie des Patienten kann dieses Verhältnis aber auch grösser oder kleiner sein. Der distale Kopfabschnitt 24 kann beispielsweise einen Durchmesser von etwa 15 mm aufweisen und der proximale Implantatabschnitt 20 am proximalen Ende 22 einen Durchmesser von etwa 9 mm.

Die Länge des proximalen Implantatabschnitts 20 ist vorliegend gut viermal so gross wie die Länge des distalen Kopfabschnitts 24 und etwa achtmal so gross wie die Länge des Übergangsabschnitts 28. Der proximale Implantatabschnitt 20 kann beispielsweise eine Länge von etwa 56 mm aufweisen, der distale Kopfabschnitt 24 eine Länge von etwa 14 mm und der Übergangsabschnitt 28 eine Länge von etwa 7 mm.

Der Kopfteil 14 besteht vorzugsweise aus Titan oder einer Titanlegierung. Er ist am distalen Kopfabschnitt 24 des Schafts 12 befestigbar und weist einen im Wesentlichen quaderförmigen Körper 36 mit abgerundeten Ecken und Kanten mit möglichst grossen Radien - beispielsweise Radien von ca. 14 mm - auf. Der Körper ist gegenüber der Längsrichtung L geneigt.

Der Kopfteil 14 weist integral mit dem Körper 36 einen sich verjüngenden Übergangsabschnitt 38 mit einer proximal angeformten, ringförmigen Stützfläche 40 auf. Der Übergangsabschnitt 38 ähnelt einem schräggestellten Pyramidenstumpf mit abgerundeten Kanten oder einem schiefen Kegelstumpf. Die Stützfläche 40 verläuft im Wesentlichen senkrecht zur Längsrichtung L und ist rotationssymmetrisch dazu. Die Stützfläche 40 ist dazu bestimmt, im implantierten Zustand der Endoprothese 10 eine distale Endfläche des Femurs zu stützen (vgl. Fig. 4) .

Gegenüber der ringförmige Stützfläche 40 vorstehend, weist der Kopfteil 14 einen integralen Verbindungszapfen 42 mit einem freien Zapfenende 44 und einer Basis 46 auf, wobei letztere innerhalb der ringförmigen Stützfläche 40 und in derselben Ebene liegt. Der gezeigte Verbindungszapfen 42 ist von der Basis 46 zum freien Zapfenende 44 hin konisch verjüngt und ist rotationssymmetrisch entlang der Längsrichtung L angeordnet.

Zur Verbindung von Kopfteil 14 und Schaft 12 wird der Verbindungszapfen 42 mit dem freien Zapfenende 44 voran in die distale Ausnehmung (vgl. Fig. 2b) des distale Kopfabschnitts 24 des Schafts 12 eingesteckt. Dabei wird ein Passsitz in Form eines Kegelsitzes ausgebildet. Dazu weist der Verbindungszapfen 42 eine als Passsitzabschnitt dienende, aussen liegende Mantelfläche 48 auf.

Auf der dem Verbindungszapfen 42 abgewandten Seite weist der Körper 36 eine Tragfläche 50 auf, welche gegenüber der Stützfläche 40 um einen Winkel von ca. 6-9° geneigt ist. Die Tragfläche 50 liegt im implantierten Zustand auf der Beinprothese auf.

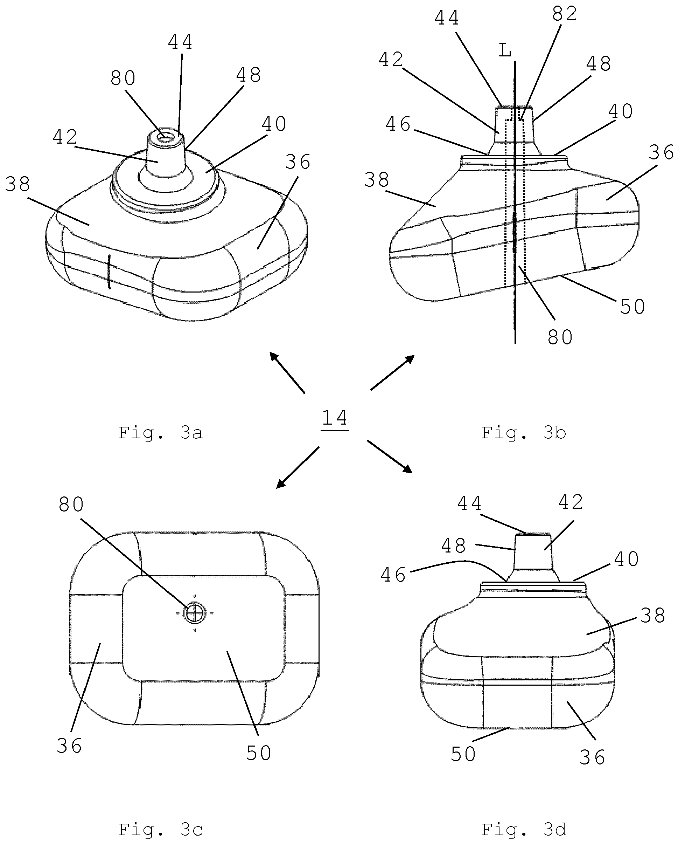

Die Schraube 16 dient zur Befestigung des Kopfteils 14 am Schaft 12 und besteht vorzugsweise aus Titan oder einer Titanlegierung. Sie umfasst einen zylindrischen Schraubenkopf 54 und einen länglichen Gewindeabschnitt 56. Zur Befestigung des Kopfteils 14 am Schaft 12 wird die Schraube 16 durch eine Öffnung im Kopfteil 14 eingeführt und mit einem Innengewinde am Schaft 12 in Eingriff gebracht. Dabei wird der Schraubenkopf 54 an einer Schulter innerhalb der Öffnung im Kopfteil 14 in Anschlag gebracht (vgl. Beschreibung zu Fig. 3a-3d).

Die Figuren 2a und 2b zeigen eine Seitenansicht bzw. einen Längsschnitt A-A des Schafts 12 aus Figur 1.

Der distale Kopfabschnitt 24 weist eine zum distalen Ende 26 hin offene, distale Ausnehmung 60 mit einer als Passsitzabschnitt dienenden, innen liegenden Mantelfläche 62 auf. Die distale Ausnehmung 60 ist rotationssymmetrisch zur Längsachse des Schafts 12. Die innen liegenden Mantelfläche 62 ist in Richtung zum proximalen Ende 22 des Schafts 12 konisch verjüngt - vorliegend weist die Mantelfläche 62 einen Konuswinkel von ca. 6° auf - und ist dazu bestimmt, mit der aussen liegenden Mantelfläche 48 des Verbindungszapfens 42 (siehe Fig. 1) einen Kegelsitz auszubilden, was zu einer sicheren Befestigung und optimalen Kräfte- und Drehmomentübertragung führt. Dabei liegt das freie Schaftende 44 nicht am Boden 64 der distalen Ausnehmung 60 an, so dass ausschliesslich der Kegelsitz tragend ist.

Am proximalen Ende der innen liegenden Mantelfläche 62 weist die distale Ausnehmung 60 einen ringförmigen Boden 64 auf. Vom Boden 64 in Richtung zum proximalen Ende 22 erstreckt sich eine zentrale Schraubenöffnung 66 zur Aufnahme der Schraube 16. Die zentrale Schraubenöffnung 66 ist im Wesentlichen sacklockförmig und weist ein zum Aussengewinde des Gewindeabschnitts 56 der Schraube 16 korrespondierendes Innengewinde auf.

Der distale Kopfabschnitt 24 ist im Wesentlichen kreiszylinderförmig, während der Übergangsabschnitt 28 und der proximale Implantatabschnitt 20 in Richtung zum proximalen Ende 22 verjüngt ausgestaltet sind. Vorliegend beträgt der Winkel zwischen der Längsrichtung L und einer Mantellinie M des proximalen Implantatabschnitts 20 ca. 1-2°, während der Übergangsabschnitt 28 deutlich steiler ist.

An seinem proximalen Ende 22 weist der Implantatabschnitt 20 eine Halteöffnung 70 auf. Die Halteöffnung 70 dient dazu, den Schaft 12 zu fixieren, während die Schraube 16 angezogen wird. Dazu weist die Halteöffnung 70 ein geeignetes Profil auf, beispielsweise einen Schlitz, Innenmehrkant oder Torx, insbesondere wie vorliegend gezeigt einen Innensechskant, auf.

Die Figuren 3a bis 3d zeigen den Kopfteil 14 aus Figur 1 in verschiedenen Ansichten.

Nebst den oben in Zusammenhang mit Figur 1 beschriebenen Merkmalen weist der Kopfteil 14 in der Längsrichtung L eine in Figur 3b gestrichelt eingezeichnete, durchgehende Öffnung 80 auf, die von der Stützfläche 40 bis zum freien Zapfenende 44 verläuft. Die Öffnung 80 erlaubt das Einführen der Schraube 16. Im Bereich des freien Zapfenendes 44 weist die Öffnung 80 eine Schulter 82 auf, die als Anschlagfläche für den Schraubenkopf 54 dient.

Die Stützfläche 40 ist eben, ringförmig und weist einen Aussendurchmesser von ca. 34 mm auf. Je nach Grösse der distalen Endfläche des Femurstumpfes (vgl. Fig. 4) kann aber auch eine Stützfläche mit einem grösseren oder kleineren Aussendurchmesser sinnvoll sein: Der Aussendurchmesser der Stützfläche 40 sollte nur leicht grösser sein als der Aussendurchmesser der distalen Endfläche des Femurstumpfes.

Die Tragfläche 50 ist eben und rechteckig mit abgerundeten Ecken. Sie weist ein Seitenverhältnis von ca. 1.3:1 auf. Geeignete Masse für Länge und Breite sind beispielsweise 80 und 62 mm. Diese Masse variieren aber natürlich von Patient zu Patient.

Der Verbindungszapfen 42 ist relativ zur Tragfläche 50 seitlich versetzt in Richtung zu einer Ecke hin verschoben.

Auch die Gesamthöhe des Kopfteils 14, in der Längsrichtung L gemessen, muss an die anatomischen Verhältnisse beim Patienten angepasst werden. Eine geeignete Höhe ist beispielsweise ca. 63 mm (vom freien Zapfenende 44 bis zum tiefsten Punkt des Körpers 36). Dabei müssen aber natürlich insbesondere auch die Höhen des Verbindungszapfens 42, des Übergangsabschnitts 38 und des Körpers 36 einzeln an die Anatomie angepasst sein. Der Verbindungszapfen 42 kann beispielsweise eine Höhe von ca. 16 mm aufweisen.

Figur 4 zeigt eine teiltransparente Seitenansicht eines Femurstumpfes 90 mit einer darin implantierten Endoprothese 10 gemäss Figur 1.

Der Schaft 12 befindet sich in der Markhöhle 92 des Femurstumpfes 90 und ist dort befestigt, beispielsweise einzementiert. Durch die länglichen Nuten 30 im Bereich des proximalen Implantatabschnitts 20 wird eine Rotation des Schafts 12 relativ zum Femurstumpf 90 verhindert. Die durch den Schaft 12 definierte Längsrichtung L entspricht im Wesentlichen der Längsrichtung des Femurstumpfes 90.

Die ringförmige Stützfläche 40 liegt an der distalen Endfläche 94 des Femurstumpfes 90 an und stützt diese. Die Tragfläche 50 des Kopfteils 14 ist gegenüber der Stützfläche 40 um einen Winkel von etwa 6-9° geneigt. Je nach Anatomie des Patienten kann aber auch ein grösserer oder kleinerer Winkel ideal sein.

## Patentansprüche

1. Endoprothese (10) für einen Röhrenknochen, insbesondere für einen Femurstumpf (90), mit einer Knochenwand und einer Markhöhle (92), aufweisend:
einen eine Längsrichtung L definierenden, wenigstens annähernd geraden Schaft (12) mit einem ein proximales Ende (22) aufweisenden, proximalen Implantatabschnitt (20) und einem ein distales Ende (26) aufweisenden, distalen Kopfabschnitt (24), wobei der Schaft (12) dazu bestimmt ist, in die Markhöhle (92) des Röhrenknochens eingesetzt zu werden;
einen am distalen Kopfabschnitt (24) des Schafts (12) befestigbaren Kopfteil (14) mit einer am Kopfteil angeformten Stützfläche (40), die dazu bestimmt ist, im implantierten Zustand der Endoprothese eine distale Endfläche (94) des Röhrenknochens zu stützen; und
ein Befestigungselement zur Befestigung des Kopfteils (14) am Schaft (12), wobei
der distale Kopfabschnitt (24) des Schafts (12) eine zum distalen Ende (26) hin offene, distale Ausnehmung (60) mit einem Boden (64) aufweist und
der Kopfteil (14) einen gegenüber der Stützfläche (40) vorstehenden, integralen Verbindungszapfen (42) mit einer Basis (46) und einem freien Zapfenende (44) aufweist, welcher dazu bestimmt ist, zur Verbindung von Kopfteil (14) und Schaft (12) mit dem freien Ende (44) voran in die distale Ausnehmung (60) des Schafts (12) eingesteckt zu werden, wobei
der Kopfteil (14) eine in Längsrichtung L verlaufende, durchgehende Öffnung (80) mit einer Schulter (82) aufweist und der Schaft (12) eine vom Boden (64) der distalen Ausnehmung (60) ausgehende, in Längsrichtung L verlaufende, sacklochförmige Vertiefung aufweist, wobei das Befestigungselement (16) in die Öffnung (80) des Kopfteils (14) einsetzbar ist und im montierten Zustand mit der sacklochförmigen Vertiefung zusammenwirkt,
**dadurch gekennzeichnet, dass** die Schulter im Bereich des freien Zapfenendes (44) angeordnet ist.

2. Endoprothese nach Anspruch 1, **dadurch gekennzeichnet, dass** die Ausnehmung (60) des Schafts (12) einen Passsitzabschnitt mit einer innen liegenden Mantelfläche (62) aufweist und der Verbindungszapfen (42) des Kopfteils (14) einen korrespondierenden Passsitzabschnitt mit einer aussen liegenden Mantelfläche (48) aufweist, wobei die beiden Mantelflächen (62, 48) dazu bestimmt sind, im montierten Zustand aneinander anzuliegen, um einen Passsitz auszubilden.

3. Endoprothese nach Anspruch 2, **dadurch gekennzeichnet, dass** die innen liegende Mantelfläche (62) der Ausnehmung des Schaftes (12) in Richtung zum Boden (64) der Ausnehmung (60) konisch verjüngt ausgebildet ist und die aussen liegende Mantelfläche (48) des Verbindungszapfens (42) in Richtung zum freien Ende (44) des Verbindungszapfens (42) konisch verjüngt ausgebildet ist, um den Passsitz als Kegelsitz auszubilden.

4. Endoprothese nach Anspruch 3, **dadurch gekennzeichnet, dass** die innen liegende Mantelfläche (62) und die aussen liegende Mantelfläche (48) einen Konuswinkel von 2° bis 12°, vorzugsweise von 6°, aufweisen.

5. Endoprothese nach einem der Ansprüche 2 bis 4, **gekennzeichnet durch** einen zwischen dem Passsitzabschnitt des Kopfteils (14) und der Basis (46) des Verbindungszapfens (42) angeordneten, konusförmigen Abschnitt.

6. Endoprothese nach einem der Ansprüche 1 bis 5, **dadurch gekennzeichnet, dass** als Befestigungselement eine Schraube (16) verwendet wird.

7. Endoprothese nach Anspruch 6, **dadurch gekennzeichnet, dass** die Schraube (16) im montierten Zustand in ein Innengewinde der sacklochförmigen Vertiefung des Kopfteils (14) eingreift.

8. Endoprothese nach Anspruch 7, **gekennzeichnet durch** eine Abdeckhülse, welche dazu bestimmt ist, im montierten Zustand in die Öffnung (80) des Kopfteils (14) eingesetzt zu sein und das Befestigungselement vom umliegenden Gewebe abzutrennen.

9. Endoprothese nach einem der Ansprüche 1 bis 8, **dadurch gekennzeichnet, dass** der proximale Implantatabschnitt (20) in Richtung zum proximalen Ende (22) sich verjüngend ausgebildet ist und vorzugsweise mit mindestens einem Verdrehsicherungselement (30) versehen ist, insbesondere mit einer oder mehreren in Längsrichtung L verlaufenden Vertiefungen, Nuten (30), Anfasungen oder seitlich abstehenden Rippen.

10. Endoprothese nach einem der Ansprüche 1 bis 9, **dadurch gekennzeichnet, dass** sich die Stützfläche (40) des Kopfteils (14) wenigstens annährend ringförmig von der Basis (46) des Verbindungszapfens (42) radial auswärts erstreckt und vorzugsweise mit einer gerippten, vorzugsweise metallischen, Oberflächenstruktur versehen ist.

11. Endoprothese nach einem der Ansprüche 1 bis 10, **dadurch gekennzeichnet, dass** der Kopfteil (14) auf der dem Verbindungszapfen (42) abgewandten Seite eine vorzugsweise wenigstens annährend rechteckige und vorzugsweise wenigstens annährend ebene Tragfläche (50) aufweist, die in radialer Richtung über die Stützfläche (40) hinausragt.

12. Endoprothese nach Anspruch 11, **dadurch gekennzeichnet, dass** die Tragfläche (50) gegenüber der Stützfläche (40) um einen Winkel von 4 bis 12°, besonders bevorzugt um einen Winkel von 6 bis 9°, geneigt ist.

13. Endoprothese-Set enthaltend eine Anzahl von unterschiedlichen Schäften und Kopfteilen nach einem der Ansprüche 1 bis 12, wobei die Ausnehmungen und Verbindungszapfen derart geformt sind, dass jeder Schaft mit jedem Kopfteil verbunden werden kann.

## Claims

1. Endoprosthesis (10) for a long bone, in particular for a femoral stump (90), having a bone wall and a medullary cavity (92), having:
an at least approximately straight shaft (12), defining a longitudinal direction L, with a proximal implant portion (20) having a proximal end (22), and a distal head portion (24) having a distal end (26), wherein the shaft (12) is intended to be inserted into the medullary cavity (92) of the long bone;
a head part (14), able to be fastened to the distal head portion (24) of the shaft (12), with a supporting surface (40) integrally formed on the head part, said supporting surface (40) being intended to support a distal end face (94) of the long bone in the implanted state of the endoprosthesis; and
a fastening element for fastening the head part (14) to the shaft (12), wherein
the distal head portion (24) of the shaft (12) has a distal recess (60), open towards the distal end (26), with a bottom (64), and
the head part (14) has an integral connecting peg (42), protruding from the supporting surface (40), with a base (46) and a free peg end (44), said connecting peg (42) being intended, in order to connect the head part (14) and shaft (12), to be plugged, with the free end (44) first, into the distal recess (60) in the shaft (12), wherein
the head part (14) has a through-opening (80), extending in the longitudinal direction L, with a shoulder (82), and the shaft (12) has a depression, in the form of a blind hole, extending in the longitudinal direction L from the bottom (64) of the distal recess (60), wherein the fastening element (16) is insertable into the opening (80) in the head part (14) and cooperates with the depression in the form of a blind hole in the assembled state,
**characterized in that** the shoulder is arranged in the region of the free peg end (44).

2. Endoprosthesis according to Claim 1, **characterized in that** the recess (60) in the shaft (12) has a close-fit portion with an internal lateral surface (62), and the connecting peg (42) of the head part (14) has a corresponding close-fit portion with an external lateral surface (48), wherein the two lateral surfaces (62, 48) are intended to bear against one another in the assembled state, in order to form a close fit.

3. Endoprosthesis according to Claim 2, **characterized in that** the internal lateral surface (62) of the recess in the shaft (12) is formed in a manner tapering conically in the direction of the bottom (64) of the recess (60), and the external lateral surface (48) of the connecting peg (42) is formed in a manner tapering conically in the direction of the free end (44) of the connecting peg (42), in order to form the close fit as a taper fit.

4. Endoprosthesis according to Claim 3, **characterized in that** the internal lateral surface (62) and the external lateral surface (48) have a cone angle of 2° to 12°, preferably of 6°.

5. Endoprosthesis according to one of Claims 2 to 4, **characterized by** a conical portion arranged between the close-fit portion of the head part (14) and the base (46) of the connecting peg (42).

6. Endoprosthesis according to one of Claims 1 to 5, **characterized in that** a screw (16) is used as fastening element.

7. Endoprosthesis according to Claim 6, **characterized in that** the screw (16) engages, in the assembled state, in an internal thread of the depression in the form of a blind hole in the head part (14).

8. Endoprosthesis according to Claim 7, **characterized by** a cover sleeve that is intended to have been inserted into the opening (80) in the head part (14) in the assembled state and to separate the fastening element from surrounding tissue.

9. Endoprosthesis according to one of Claims 1 to 8, **characterized in that** the proximal implant portion (20) is formed in a manner tapering in the direction of the proximal end (22) and is provided preferably with at least one twist prevention element (30), in particular with one or more depressions, grooves (30), chamfers or laterally protruding ribs extending in the longitudinal direction L.

10. Endoprosthesis according to one of Claims 1 to 9, **characterized in that** the supporting surface (40) of the head part (14) extends radially outwards at least approximately in an annular manner from the base (46) of the connecting peg (42) and is provided preferably with a ribbed, preferably metallic, surface structure.

11. Endoprosthesis according to one of Claims 1 to 10, **characterized in that** the head part (14) has, on the side remote from the connecting peg (42), a preferably at least approximately rectangular and preferably at least approximately planar bearing surface (50) that projects in a radial direction beyond the supporting surface (40).

12. Endoprosthesis according to Claim 11, **characterized in that** the bearing surface (50) is inclined at an angle of 4 to 12°, particularly preferably at an angle of 6 to 9°, to the supporting surface (40).

13. Endoprosthesis set containing a number of different shafts and head parts according to one of Claims 1 to 12, **wherein** the recesses and connecting pegs are shaped such that each shaft can be connected to each head part.

## Revendications

1. Endoprothèse (10) pour un os long, en particulier pour un moignon fémoral (90), comprenant une paroi osseuse et une cavité médullaire (92), présentant :
une tige (12) au moins approximativement droite, définissant une direction longitudinale L avec une portion d'implant proximale (20) présentant une extrémité proximale (22) et une portion de tête distale (24), présentant une extrémité distale (26), la tige (12) étant prévue pour être insérée dans la cavité médullaire (92) de l'os long ;
une partie de tête (14) pouvant être fixée à la portion de tête distale (24) de la tige (12), avec une surface de support (40) façonnée sur la partie de tête, qui est prévue, dans l'état implanté de l'endoprothèse, pour supporter une surface d'extrémité distale (94) de l'os long ; et
un élément de fixation pour la fixation de la partie de tête (14) à la tige (12),
la portion de tête distale (24) de la tige (12) présentant un évidement distal (60) ouvert vers l'extrémité distale (26), avec un fond (64) et
la partie de tête (14) présentant un tourillon de liaison intégral (42) faisant saillie par rapport à la surface de support (40), avec une base (46) et une extrémité de tourillon libre (44), qui est prévu pour relier la partie de tête (14) et la tige (12) avec l'extrémité libre (44) en avant dans l'évidement distal (60) de la tige (12),
la partie de tête (14) présentant une ouverture traversante (80) s'étendant dans la direction longitudinale L avec un épaulement (82) et la tige (12) présentant un renfoncement en forme de trou borgne s'étendant dans la direction longitudinale L depuis le fond (64) de l'évidement distal (60), l'élément de fixation (16) pouvant être inséré dans l'ouverture (80) de la partie de tête (14) et coopérant dans l'état monté avec le renfoncement en forme de trou borgne,
**caractérisée en ce que** l'épaulement est disposé dans la région de l'extrémité de tourillon libre (44).

2. Endoprothèse selon la revendication 1, **caractérisée en ce que** l'évidement (60) de la tige (12) présente une portion d'ajustement serré avec une surface d'enveloppe (62) située à l'intérieur et le tourillon de liaison (42) de la partie de tête (14) présente une portion d'ajustement serré correspondant avec une surface d'enveloppe (48) située à l'extérieur, les deux surfaces d'enveloppe (62, 48) étant prévues pour s'appliquer l'une contre l'autre dans l'état monté, afin de constituer un ajustement serré.

3. Endoprothèse selon la revendication 2, **caractérisée en ce que** la surface d'enveloppe située à l'intérieur (62) de l'évidement de la tige (12) est réalisée de manière à se rétrécir sous forme conique dans la direction du fond (64) de l'évidement (60), et la surface d'enveloppe située à l'extérieur (48) du tourillon de liaison (42) est réalisée de manière à se rétrécir sous forme conique dans la direction de l'extrémité libre (44) du tourillon de liaison (32) afin de constituer l'ajustement serré sous forme de siège conique.

4. Endoprothèse selon la revendication 3, **caractérisée en ce que** la surface d'enveloppe située à l'intérieur (62) et la surface d'enveloppe située à l'extérieur (48) présentent un angle de conicité de 2° à 12°, de préférence de 6°.

5. Endoprothèse selon l'une quelconque des revendications 2 à 4, **caractérisée par** une portion de forme conique disposée entre la portion d'ajustement serré de la partie de tête (14) et la base (46) du tourillon de liaison (42).

6. Endoprothèse selon l'une quelconque des revendications 1 à 5, **caractérisée en ce que** l'on utilise une vis (16) en tant qu'élément de fixation.

7. Endoprothèse selon la revendication 6, **caractérisée en ce que** la vis (16), dans l'état monté, s'engage dans un filetage intérieur du renfoncement en forme de trou borgne de la partie de tête (14).

8. Endoprothèse selon la revendication 7, **caractérisée par** une douille de recouvrement qui est prévue pour être insérée dans l'état monté dans l'ouverture (80) de la partie de tête (14) et pour séparer l'élément de fixation du tissu environnant.

9. Endoprothèse selon l'une quelconque des revendications 1 à 8, **caractérisée en ce que** la portion d'implant proximale (20) est réalisée de manière à se rétrécir dans la direction de l'extrémité proximale (22) et de préférence est pourvue d'au moins un élément de fixation en rotation (30), en particulier d'un ou plusieurs renfoncements, rainures (30), biseaux s'étendant dans la direction longitudinale L ou de nervures saillant latéralement.

10. Endoprothèse selon l'une quelconque des revendications 1 à 9, **caractérisée en ce que** la surface de support (40) de la partie de tête (14) s'étend au moins approximativement sous forme annulaire depuis la base (46) du tourillon de liaison (42) radialement vers l'extérieur et est de préférence pourvue d'une structure de surface nervurée, de préférence métallique.

11. Endoprothèse selon l'une quelconque des revendications 1 à 10, **caractérisée en ce que** la partie de tête (14), du côté opposé au tourillon de liaison (42), présente une surface porteuse (50) de préférence au moins approximativement rectangulaire et de préférence au moins approximativement plane, qui fait saillie dans la direction radiale au-delà de la surface de support (40).

12. Endoprothèse selon la revendication 11, **caractérisée en ce que** la surface porteuse (50) est inclinée par rapport à la surface de support (40) d'un angle de 4 à 12°, particulièrement préférablement d'un angle de 6 à 9°.

13. Ensemble d'endoprothèse comprenant une pluralité de tiges et de parties de tête différentes selon l'une quelconque des revendications 1 à 12, dans lequel les évidements et les tourillons de liaison sont formés de telle sorte que chaque tige puisse être connectée à chaque partie de tête.
